# EUROPEAN PATENT APPLICATION

(11) **EP 4 212 239 A1**
(43) Date of publication of application: **19.07.2023**
(21) Application number: 22151570.3
(22) Date of filing: 14.01.2022
(51) Int. Cl.: B01J 13/14, A23K 40/30, A23L 27/00, A61K 8/11, A61Q 5/00, A61Q 19/00, B01J 13/16, C11D 3/50, A61K 9/48

(54) **BIODEGRADABLE PREPOLYMER MICROCAPSULES**

(71) Applicant: International Flavors & Fragrances Inc., New York, NY 10019 (US)
(72) Inventor: SASAKI, Takashi, Union Beach, NJ, 07735 (US); CHIN, Ryan, Union Beach, NJ, 07735 (US); GABBARD, Ronald, Union Beach, NJ, 07735 (US); HACKENBERG, Jason, Union Beach, NJ, 07735 (US); LEI, Yabin, Union Beach, NJ, 07735 (US); LU, Helen, Wilmington, DE, 19803 (US); POPPLEWELL, Lewis Michael, Union Beach, NJ, 07735 (US); SAUCA, Sorin Nicolae, 12580 Benicarlo (ES); TRAN, Tram, Union Beach, NJ, 07735 (US); WIELAND, Julie Ann, Union Beach, NJ, 07735 (US); YOUNG, Timothy, Union Beach, NJ, 07735 (US)
(74) Representative: DuPont EMEA

(57) **Abstract**

Biodegradable core-shell microcapsules prepared with a crosslinked biodegradable, isocyanate-terminated prepolymer are provided, as is a process for preparing the biodegradable core-shell microcapsules and consumer products containing the same.

## Description

### BACKGROUND OF THE INVENTION

Current commercial fragrance capsules including melamine-formaldehyde and polyurea are made from synthetic materials, which are not readily biodegradable. This is an issue for several reasons. Firstly, consumers are demanding more environmentally friendly products. Secondly, due to new regulations, for example from the European Chemicals Agency (ECHA), there will be a ban on the use of microplastics in a variety of consumer goods products (e.g., cosmetics, detergents, etc.). As a result, there is an ever-increasing demand for fragrance delivery technologies where the capsule wall material is more biodegradable and/or sustainable, and will satisfy the requirements set out in any currently existing and newly proposed regulations banning the use of microplastics.

While methods to include biopolymer composite materials in the wall of microcapsules have been described, polyisocyanates are first dissolved in the oil phase and then emulsified together with an aqueous phase containing polyamines, polyelectrolytes or biopolymers to react at the oil-water interface. See US 8,663,690 B2 (Rice University); US 10,034,819 B2 (Firmenich); EP 3478403 A1 (Firmenich); WO 2020/195132 A1 (Fujifilm); WO 2021/116365 A1 (Henkel); WO 2021/115601 A1 (Koehler SE), GB 2029791 A (Oji Paper); WO 2021/122636 A1 (Firmenich) and WO 2021/122633 A1 (Firmenich). While the intended reaction is an interfacial polymerization, controlling the kinetics to yield a copolymer/composite is challenging and often results in self-condensation of polyisocyanate coated with a layer of unreacted material originally intended for the interfacial polymerization. However, the degree or rate of biodegradability of these capsules is not always described.

Even assuming, arguendo, that the microcapsules disclosed herein above may pass any current regulations (*e*.*g*., the OECD301F or OECD310 tests), they may still not be 'truly' biodegradable and fully ECHA compliant. By that we mean that the microcapsules may not satisfy the requirements under the proposed ECHA regulations that materials forming the microcapsule walls are not to be considered a "blend" of biodegradable materials (*i*.*e*., biopolymers) and non-biodegradable materials. Alternatively, the microcapsule walls are considered a blend then all components of the blend would also meet the requirements for biodegradability (*e*.*g*., minimally at least the OECD301F or OECD310 tests).

Polyurea/polyurethane co-polymers and composites prepared from the reaction of polyisocyanates and biodegradable polyelectrolytes/biopolymers such as gelatin and chitosan have been described in terms of mechanical properties and functional benefits (Bertoldo et al. (2007) Macromol. Biosci. 7:328-338; Gallego (2013) Molecules 18:6532-6549; Koebel et al. (2016) J. Sol-Gel Sci. Technol. 79:308-318). However, use of the same in the encapsulation of active materials has not been described.

Accordingly, microcapsules composed of sustainable, and/or biodegradable materials, which are chemically stable and/or deliver active materials such as fragrances in consumer products are still needed in the industry. Preferably, the materials used to form the microcapsules do not form a blend of biodegradable materials and non-biodegradable materials. Alternatively, if the materials used to form the microcapsule walls do form a blend, then not a significant amount (*i*.*e*., ≤ 10%, ≤ 5%, ≤ 3%, ≤ 1% or ≤ 0.5%) of non-biodegradable materials or all components of the blend would also meet the requirements for biodegradability. The microcapsules preferably also provide acceptable fragrance profile and performance in use. The present invention satisfies this and other needs in the industry.

### SUMMARY OF THE INVENTION

This invention is based on the discovery that biodegradable and/or sustainable microcapsule shells can be prepared with prepolymers of isocyanate-terminated biodegradable polymers.

Accordingly, this invention provides biodegradable core-shell microcapsules comprising (a) an isocyanate-biodegradable polymer shell comprising the reaction product of a biodegradable, isocyanate-terminated prepolymer with a crosslinker and optionally a polyelectrolyte emulsifier under aqueous conditions; and (b) a core comprising an active material; wherein the microcapsule shell has a biodegradation rate of at least 20%, 30%, 40%, 50%, 60%, 65%, 70%, 75%, 80%, 85%, 90%, 95% or 98%, within 60 days according to OECD301F or OECD310.

In some aspects, the biodegradable, isocyanate-terminated prepolymer is prepared with gelatin, collagen, chitosan, modified guar, modified glucan, gum Arabic (gum acacia), modified gum Arabic (modified gum acacia), protein, hydrolyzed proteins, fermented proteins, hydrophobin, enzymes, partially neutralized citric acid ester, alginate, carrageenan, pectin, modified starch, or modified cellulose. In other aspects, the crosslinker is an oxidized sugar, preferably the oxidized sugar comprises glucose, glucosamine, sucrose, maltose, lactose, maltodextrin, cyclodextrin, polysaccharide or a hydrolyzed polysaccharide.

In further aspects, wherein: (a) obtained slurry of the biodegradable core-shell microcapsules has a level of self-condensed polyisocyanate that is below 10%, below 5%, below 3%, below 1% or below 0.5%, relative to total weight of polyisocyanate used to form wall of microcapsules; (b) materials used to form wall of the microcapsules do not form a blend of biodegradable materials and non-biodegradable materials; (c) materials used to form wall of the microcapsules forms a blend of biodegradable materials and non-biodegradable materials, wherein the levels of the non-biodegradable materials are below 10%, below 5%, below 3%, below 1% or below 0.5%, relative to weight of the microcapsules; or (d) materials used to form the microcapsules forms a blend of biodegradable materials and non-biodegradable materials, wherein the biodegradation rate of all components of the blend is at least 20%, 30%,40%, 50%, 60%, 65%, 70%, 75%, 80%, 85%, 90%, 95% or 98%, within 60 days according to OECD301F or OECD310.

In further aspects, the isocyanate-biodegradable polymer shell is highly crosslinked, preferably the average molecular weight of crosslinked prepolymer and polyelectrolyte emulsifier is at least 2, at least 4, at least 10 of the average molecular weight of non-crosslinked prepolymer and polyelectrolyte emulsifier, as determined by size exclusion chromatography. In yet other aspects, the polyelectrolyte emulsifier comprises gelatin, collagen, modified guar, modified glucan, gum Arabic (gum acacia), modified gum Arabic (modified gum acacia), protein, hydrolyzed proteins, fermented proteins, hydrophobin, enzymes, partially neutralized citric acid ester, alginate, carrageenan, pectin, modified starch, or modified cellulose.

An active material suitable for encapsulation may include a fragrance, flavor, agricultural active, pharmaceutical active, nutraceutical active, animal nutrition active, food active, microbio active, malodor counteractant, and/or cosmetic active, preferably a fragrance. The invention is also based, *inter alia*, on the discovery of High Performance fragrance ingredients composed of certain Ultra High-Impact fragrance ingredients and High-Impact fragrance ingredients to deliver improved perceived intensity, perceived longevity and/or perceived fidelity of the fragrance profile at the various "touch points" (*e*.*g*., opening a fabric conditioner container, damp clothes upon opening a washing machine after washing laundry, opening a laundry dryer after drying laundry, drying clothes on drying frame and wearing laundered clothes) associated with the laundry experience. Preferably, the fragrance comprises at least one, at least two, at least three, at least four, at least five, at least six, at least seven, at least eight, at least nine or at least ten High Performance fragrance ingredients comprising Ultra High-Impact fragrance ingredients as listed in Table 1 and High-Impact fragrance ingredients as listed in Table 2, and optionally at least one additional fragrance ingredient.

This invention also provides a process for preparing biodegradable core-shell microcapsules by (a) reacting, under anhydrous conditions, a polyisocyanate dissolved in a solution with a biodegradable polymer, preferably in the presence of a catalyst, to form an isocyanate-terminated prepolymer, preferably the solution comprises (i) a water-soluble solvent, (ii) a water-soluble solvent comprising an active material or a portion thereof, or (iii) an active material, preferably the active material comprises a fragrance, flavor, agricultural active, pharmaceutical active, nutraceutical active, animal nutrition active, food active, microbio active, malodor counteractant, and/or cosmetic active, more preferably a fragrance; (b) emulsifying the isocyanate-terminated prepolymer with an aqueous solution to form an emulsion, preferably said aqueous solution comprises a polyelectrolyte emulsifier; and (c) crosslinking the isocyanate-terminated prepolymer to form biodegradable core-shell microcapsules; preferably the microcapsule shell has a biodegradation rate of at least 20%, 30%, 40%, 50%, 60%, 65%, 70%, 75%, 80%, 85%, 90%, 95% or 98%, within 60 days according to OECD301F or OECD310.

In some aspects, the biodegradable polymer is an amino-, hydroxyl- or carboxyl-containing biodegradable polymer, preferably the biodegradable polymer comprises gelatin, collagen, chitosan, modified guar, modified glucan, gum Arabic (gum acacia), modified gum Arabic (modified gum acacia), protein, hydrolyzed proteins, fermented proteins, hydrophobin, enzymes, partially neutralized citric acid ester, alginate, carrageenan, pectin, modified starch, or modified cellulose. In other aspects, the polyelectrolyte emulsifier comprises gelatin, collagen, modified guar, modified glucan, gum Arabic (gum acacia), modified gum Arabic (modified gum acacia), protein, hydrolyzed proteins, fermented proteins, hydrophobin, partially neutralized citric acid ester, alginate, carrageenan, pectin, modified starch, or modified cellulose. In further aspects, the isocyanate-terminated prepolymer and/or the polyelectrolyte emulsifier is crosslinked with an oxidized sugar, preferably the sugar comprises glucose, glucosamine, sucrose, maltose, lactose, maltodextrin, cyclodextrin, polysaccharide or a hydrolyzed polysaccharide.

Biodegradable core-shell microcapsules obtainable by the process are also provided as is a consumer product including biodegradable core-shell microcapsules. Examples of consumer products include a fabric softener, fabric conditioner, detergent, scent booster, fabric refresher spray, body wash, body soap, shampoo, hair conditioner, body spray, hair refresher spray, hair dye, hair moisturizer, skin moisturizer, hair treatment, skin treatment, antiperspirant, deodorant, insect repellant, candle, surface cleaner, bathroom cleaner, bleach, cat litter, refresher spray or pesticide.

This invention further provides a biodegradable, isocyanate-terminated prepolymer formed under anhydrous conditions in the presence of a catalyst for use in preparing core-shell microcapsules, wherein the prepolymer is the reaction product of: (a) a polyisocyanate dissolved in an active material and optionally a solvent; and (b) an amino-, hydroxyl- or carboxyl-containing biodegradable polymer, preferably the biodegradable polymer comprises gelatin, collagen, chitosan, modified guar, modified glucan, gum Arabic (gum acacia), modified gum Arabic (modified gum acacia), protein, hydrolyzed proteins, fermented proteins, hydrophobin, partially neutralized citric acid ester, alginate, carrageenan, pectin, modified starch, or modified cellulose; wherein the prepolymer has a biodegradation rate of at least 20%, 30%, 40%, 50%, 60%, 65%, 70%, 75%, 80%, 85%, 90%, 95% or 98%, within 60 days according to OECD301F or OECD310.

The invention also provides a process for making a biodegradable, isocyanate-terminated prepolymer for use in preparing core-shell microcapsules by reacting (a) a polyisocyanate dissolved in a solution, preferably the solution comprises (i) a water-soluble solvent, (ii) a water-soluble solvent comprising an active material or a portion thereof, or (iii) an active material, preferably the active material comprises a fragrance, flavor, agricultural active, pharmaceutical active, nutraceutical active, animal nutrition active, food active, microbio active, malodor counteractant, and/or cosmetic active, more preferably a fragrance; and (b) a biodegradable polymer, preferably the biodegradable polymer is an amino-, hydroxyl- or carboxyl-containing biodegradable polymer, more preferably the biodegradable polymer comprises gelatin, collagen, chitosan, modified guar, modified glucan, gum Arabic (gum acacia), modified gum Arabic (modified gum acacia), protein, hydrolyzed proteins, fermented proteins, hydrophobin, partially neutralized citric acid ester, alginate, carrageenan, pectin, modified starch, or modified cellulose; characterized in that reaction is carried out under anhydrous conditions in the presence of a catalyst. A biodegradable, isocyanate-terminated prepolymer obtainable by the process is also encompassed by this invention. The terms "obtainable" or "obtained" are used interchangeably and does not mean to indicate that, *e*.*g*., a product must be obtained by, *e*.*g*., the sequence of steps following the term "obtained" though such a limited understanding is always included by the terms as a preferred aspect of the present invention.

All parts, percentages and proportions referred to herein and in the claims are by weight unless otherwise indicated.

The details of one or more aspects of the invention are set forth in the description below. Other features, objects, and advantages of the invention will be apparent to those skilled in the art from the detailed description and the drawings.

### DETAILED DESCRIPTION OF THE INVENTION

Conventionally, polyurea-based microcapsules are prepared by first dissolving a polyisocyanate in an oil phase and then emulsifying the oil phase with an aqueous phase containing polyamines, polyelectrolytes or biopolymers thereby promoting the reaction of the polymers at the oil-water interface. While the intended reaction is an interfacial polymerization, controlling the kinetics to yield a copolymer/composite is difficult. It has been observed that capsules prepared via this traditional method yield a blend of polyurea formed from the self-condensation of polyisocyanate coated with a layer of unreacted material originally intended for the interfacial polymerization. The issue with such capsules is that the shell, even though they may pass the OECD301F or OECD310 tests, may still not be 'truly' biodegradable, for example under proposed ECHA regulations, because it is considered a blend of biodegradable (*i*.*e*., biopolymers) and non-biodegradable materials (*i*.*e*., self-condensing polyisocyanate) and therefore the blend would not meet the biodegradability requirements.

It has now been found that when a polyelectrolyte or biopolymer is reacted with a polyisocyanate under anhydrous conditions a prepolymer is formed, which inhibits self-condensation of polyisocyanate. This prepolymer is then combined with an active material and suitable crosslinker and core-shell microcapsules are formed, which have a shell or wall composed of a covalently polymerized, polyelectrolyte/biopolymer and polyisocyanate co-polymer. Advantageously, the resulting microcapsules are pH stable, biodegradable, exhibit a high encapsulation efficiency, and provide high performance in consumer products such as fabric conditioners. Accordingly, this invention provides a biodegradable, isocyanate-terminated prepolymer and use of the same in the preparation of biodegradable core-shell microcapsules.

The biodegradable core-shell microcapsules of this invention have a core including at least one active material, and a shell composed of at least one crosslinked prepolymer. "Biodegradable" as used herein with respect to a material, such as a microcapsule shell as a whole and/or a polymer (*e*.*g*., biodegradable polymer or prepolymer) of the microcapsule shell, has no real or perceived health and/or environmental issues, and is capable of undergoing and/or does undergo physical, chemical, thermal, microbial, biological and/or UV or photo-degradation. Ideally, a microcapsule shell and/or polymer is deemed "biodegradable" when the microcapsule shell and/or polymer passes one or more of the following tests including: a respirometry biodegradation method in aquatic media, available from Organization for Economic Cooperation and Development (OECD), International Organization for Standardization (ISO) and the American Society for testing and Material (ASTM) tests including, but not limited to OECD 301F or 310 (Ready biodegradation), OECD 302 (inherent biodegradation), ISO 17556 (solid stimulation studies), ISO 14851 (fresh water stimulation studies), ISO 18830 (marine sediment stimulation studies), OECD 307 (soil stimulation studies), OECD 308 (sediment stimulation studies), and OECD 309 (water stimulation studies). Preferably, the microcapsules are readily biodegradable as determined using a respirometry biodegradation method in aquatic media, the OECD 301F or OECD 310 test. More preferably, the shell and/or prepolymer of the microcapsules are biodegradable if the shell and/or prepolymer has a biodegradation rate of at least 20%, 30%, 40%, 50%, 60%, 65%, 70%, 75%, 80%, 85%, 90%, 95% or 98%, within 60 days according to the OECD301F or OECD310 tests, or most preferably a biodegradability of at least 60% within 60 days according to OECD301F test.

As used herein, a "core-shell microcapsule," or more generically a "microcapsule" or "capsule," is a substantially spherical structure having a well-defined core and a well-defined envelope or wall or shell. The "core" is composed of any active material or material submitted to microencapsulation. The terms "wall" and "shell" are used interchangeably to denote the structure formed by the microencapsulating prepolymer surrounding the active material core being microencapsulated. In general, the wall of the microcapsule is made of a continuous, polymeric phase with an inner surface and outer surface. The inner surface is in contact with the microcapsule core. The outer surface is in contact with the environment in which the microcapsule resides, *e.g.*, a water phase, skin, or hair. Ideally, the wall protects the core against deterioration by oxygen, moisture, light, and effect of other compounds or other factors; limits the losses of volatile core materials; and releases the core material under desired conditions. In this respect, the core-shell microcapsules of this invention provide controlled release and/or diffusional release of the active material. As used herein, "controlled release" refers to retention of the active material in the core until a specified triggering condition occurs. Such triggers include, *e*.*g*., friction, swelling, a pH change, an enzyme, a change in temperature, a change in ionic strength, or a combination thereof.

As used in the context of this invention, "biodegradable core-shell microcapsules" or "a biodegradable core-shell microcapsule composition" refers to a slurry or suspension of biodegradable core-shell microcapsules produced in accordance with the methods and examples described herein. The biodegradable core-shell microcapsule composition of this invention may be used directly in a consumer product, washed, coated, dried (*e*.*g*., spray-dried) and/or combined with one or more other microcapsule compositions, active materials, and/or carrier materials.

As used herein, a "prepolymer" refers to a polymer that have been reacted to an intermediate state and can be usefully manipulated before crosslinking is completed. A "biodegradable, isocyanate-terminated prepolymer" is a prepolymer in which all or a portion of the amino, hydroxyl and/or carboxyl end groups of a biodegradable polymer have been reacted with isocyanate groups leaving isocyanate functionality at the termini of the biodegradable polymer instead of amino, hydroxyl and/or carboxyl groups. For the sake of clarity, isocyanate-terminated prepolymer means that the isocyanate group(s) react with the amino, hydroxyl and/or carboxyl groups at multiple positions along the polymer chain, including the middle of the polymer chain, to allow for cross-linking.

The term "sustainable" as used herein with respect to a material, such as microcapsule shell and/or active material (*e*.*g*., fragrance ingredients), refers to "biobased" material, which are atoms or molecules obtained from biomass, *e*.*g*., obtained from materials containing organic carbon of renewable origin. Source of such carbon can be derived from agricultural products, plants, animals, fungi, microorganisms, marine or forestry materials.

The term "water-soluble" as used herein with respect to a solvent refers to the solvent's ability to mix and form a homogenous solution with the water.

In accordance with this invention, a prepolymer is prepared by combining (a) a polyisocyanate dissolved in an active material and optionally a solvent with (b) an amino-, hydroxyl- and/or carboxyl-containing biodegradable polymer under anhydrous conditions, preferably in the presence of a catalyst. A biodegradable polymer "containing" an amino-, hydroxyl- or carboxyl-group refers to presence of amino, hydroxyl and/or carboxyl end groups being present on the biodegradable polymer, which can react with the polyisocyanate. Thus, the biodegradable polymer may alternatively be referred to as an amino-terminated, hydroxyl-terminated and/or carboxyl-terminated biodegradable polymer.

For the purposes of this invention, a "biodegradable polymer" (or "biopolymer) is a polymer obtained from a natural source (*e*.*g*., a plant, fungus, bacterium or animal) or modified biopolymer thereof. In some aspects, the biodegradable polymer used in the preparation of the microcapsules is soluble or dispersible in the oil phase. In other aspects, the biodegradable polymer is a polyelectrolyte. In certain aspects, the biodegradable polymer is a polypeptide or polysaccharide. In other aspects, the biodegradable polymer of the microcapsule wall is a single type of polymer, *e.g.*, a polypeptide or a polysaccharide. In other aspects, the biodegradable polymer of the microcapsule wall is a combination of polymers and/or types of polymers, *e*.*g*., (a) at least one polypeptide in combination with at least one polysaccharide, (b) two or more polysaccharides, or (c) two or more polypeptides.

In particular aspects, the shell of the biodegradable core-shell microcapsules includes at least one polysaccharide such as a chitosan, modified guar, modified glucan, gum Arabic (gum acacia), modified gum Arabic (modified gum acacia), alginate, carrageenan, pectin, modified starch, or modified cellulose; at least one polypeptide such as a gelatin, collagen, protein, hydrolyzed protein, fermented protein, hydrophobin, or enzyme; or a partially neutralized citric acid ester, or any combination of the above-referenced biodegradable polymer. In some aspects, the shell of the biodegradable core-shell microcapsules includes at least two, three, or four biodegradable polymers. Preferably, the biodegradable polymer is a polypeptide. In particular, the biodegradable polymer of the microcapsule shell is gelatin.

The term "polyisocyanate" as used here is a collective term for compounds containing at least two isocyanate groups in the molecule (this is understood by the person skilled in the art to mean free isocyanate groups of the general structure: R-N=C=O). The simplest form of polyisocyanates are the diisocyanates. These have the general structure O=C=N-R-N=C=O where R typically represents aliphatic, alicyclic and/or aromatic groups. In certain aspects, the polyfunctional isocyanate is an oligomeric polyisocyanate obtained from hexamethylene diisocyanate (HDI), which is a monomeric diisocyanate. In certain aspects, the polyfunctional isocyanate is an oligomeric polyisocyanate especially having a biuret, isocyanurate, allophanate, uretdione and/or oligomeric HDI structure. Exemplary polyisocyanates are sold under the tradenames TAKENATE^{®} (*e*.*g*., TAKENATE^{®} D-110N; Mitsui Chemicals), DESMODUR^{®} (Covestro), BAYHYDUR^{®} (Covestro), and LUPRANATE^{®} (BASF).

Under aqueous conditions, isocyanate functional groups preferably react with water to form -NH₂ (via carbamic acid), which then reacts with the unreacted isocyanate functional groups to form urea bonds. To inhibit this self-condensation and promote prepolymer formation, the polyisocyanate and the biodegradable polymer are combined under anhydrous conditions (*i*.*e*., oil phase). Ideally, the polyisocyanate is dissolved in an anhydrous solution and the biodegradable polymer is likewise dissolved or dispersed therein. Suitable solutions for carrying out prepolymer formation include the use of a water-soluble solvent; an anhydrous active material; or a combination thereof (*i.e*., a water-soluble solvent comprising an anhydrous active material). Exemplary anhydrous, water-soluble solvents or dispersants of use in this invention include, but are not limited to, mineral oil, dimethylsulfoxide (DMSO), dimethylformamide (DMF), benzyl benzoate, triacetin, , ethyl acetate, propylene glycol diacetate, diethyl malonate, triethyl citrate, ethyl acetoacetate, benzyl acetone, butyl carbitol acetate, 3-methyl butyl acetate, diethylene glycol monoethyl ether acetate (DGMEA), and the like or any combination thereof. Preferably, the solvent is caprylic/capric triglyceride.

Preferably, the prepolymer formation is further facilitated by the inclusion of a catalyst in the reaction. Suitable catalysts are soluble or dispersible in the anhydrous solution containing the polyisocyanate and biodegradable polymer. The catalysts are capable of catalyzing the formation of urea, urethane, and/or amide reaction products. Ideally, the concentration of catalyst is between 0.001% and 1% by weight of the reaction, preferably between 0.01% and 0.02% by weight of the reaction. Preferably, the catalysts contain at least one tertiary amine. Examples of suitable catalysts used in the preparation of the prepolymer of this invention include, but are not limited to, 1,4-diazabicylo[2,2,2]octane (DABCO), N-methylimidazole, diaminobicycloctane, 2,2'-dimorpholinodiethylether, or any combination thereof. Preferably, the catalyst is DABCO. Suitable prepolymer reaction conditions include a pH of 1 to 12, preferably 3 to 5.5 and a temperature of 25°C to 250°C, preferably 40°C to 60°C.

Formation of the prepolymer of this invention is evident by an increase in the molecular weight of the biodegradable polymer. In principle, a prepolymer molecular weight should be the molecular weight of the biodegradable polymer plus the molecular weight of the polyisocyanate capping the biodegradable polymer. For example, when using a diisocyanate, the prepolymer molecular weight would theoretically be the molecular weight of the biodegradable polymer plus two times the molecular weight of the diisocyanate. The final molecular weight of the biodegradable polymer may vary depending on the polyisocyanate, the amount/ratio of reactants, and/or conditions of the reaction. Ideally, the prepolymer has low levels of free isocyanates (*e*.*g*., less than 12%).

The core of the biodegradable core-shell microcapsules of this invention includes at least one active material, which may also serve as the medium for carrying out prepolymer formation. Accordingly, the active material is preferably anhydrous. In other aspects, the active material is hydrophobic. In further aspects, the active material is soluble or dispersible in a solvent. Ideally, the active material has a logP value (partition coefficient) of less than 2. In certain aspects, the microcapsules include at least two, three, four or more active materials in the core. Active materials that may be encapsulated in the core-shell microcapsule of this invention include, but are not limited to, fragrances, flavors, agricultural actives, pesticides, pharmaceutical actives, nutraceutical actives, animal nutrition actives, food actives, microbio actives, malodor counteractants, and/or cosmetic actives, preferably fragrances, more preferably fragrances comprising at least one, at least two, at least three, at least four, at least five, at least six, at least seven, at least eight, at least nine or at least ten High Performance fragrance ingredients comprising Ultra High-Impact fragrance ingredients as listed in Table 1 and High-Impact fragrance ingredients as listed in Table 2.

**Table 1 - Ultra High-Impact Fragrance Ingredients**

| **No.** | **Trade Name** | **CAS** |
|---|---|---|
| 1. | ALD C-11 INTRELEVEN (TT) PRG | 143-14-6 |
| 2. | ALD C-6 TOCO | 66-25-1 |
| 3. | AMBER XTREME^{™} | 476332-65-7 647828-16-8 |
| 4. | AMBERMOR EX | 6790-58-5 |
| 5. | AMBERMORKETAL | 57345-19-4 |
| 6. | AMBROCENIDE CRYST | 211299-54-6 |
| 7. | CALONE | 28940-11-6 |
| 8. | CITRYLAL BHT | 90480-35-6 147060-73-9 |
| 9. | CLONAL | 2437-25-4 |
| 10. | CREOSOL #340 | 93-51-6 |
| 11. | CRESYL ACET PARA COEUR | 140-39-6 |
| 12. | DECENAL,CIS-4 | 21662-09-9 |
| 13. | DODECENAL TRANS-2 TOCO | 20407-84-5 |
| 14. | DUPICAL TOCO | 30168-23-1 |
| 15. | DYNASCONE BHT | 56973-85-4 |
| 16. | FLORAL SUPER | 71077-31-1 |
| 17. | FRAGARONE | 16957-70-3 |
| 18. | GALBASCONE ALPHA 95 PRG | 56973-85-4 |
| 19. | GALBASCONE PRG TOCO | 56973-85-4 56973-84-3 |
| 20. | ISO BUTYL QUINOLINE | 67634-06-4 |
| 21. | ISO BUTYL THIAZOLE | 18640-74-9 |
| 22. | JAMUNATE | 7605-52-9 |
| 23. | KOUMALACTONE 10 PCT TEC FIRM | 77-93-0 |
| 24. | MANGONE CSM | 38462-22-5 |
| 25. | MELOZONE | 30772-79-3 |
| 26. | METH HEPTIN CARBONATE | 111-12-6 |
| 27. | METH LAITONE 10 PCT DPG | 110-98-5 |
| 28. | METH PHEN ETH ETHER | 3558-60-9 |
| 29. | NONADIENAL,2-TR-6-CIS TOCO | 557-48-2 |
| 30. | NONENOL,CIS-6 TOCO | 35854-86-5 |
| 31. | OPALENE TOCO | 174155-47-6 |
| 32. | ORENYLE | 29127-83-1 |
| 33. | ORRIS ALD TOCO | 60784-31-8 |
| 34. | OXANE 50 PCT TEC | 59323-76-1 59324-17-3 |
| 35. | PASSION FRUIT CMPD | 59323-76-1 |
| 36. | PHARAONE 10 PCT DPG | 110-98-5 |
| 37. | PYRAZINE 044 (METH) | 109-08-0 |
| 38. | ROSE OXIDE "L" TOCO | 16409-43-1 |
| 39. | ROSYRANE SUPER | 60335-71-9 |
| 40. | SACRAZOLE-018 | 137-00-8 |
| 41. | THIAZOLE (2-ISO PROP 4-METH) | 15679-13-7 |
| 42. | TRIDECENE-2-NITRILE | 22629-49-8 |
| 43. | TROPICALIA TOCO | 10138-32-6 |
| 44. | VIONIL NEAT | 67019-89-0 |
| 45. | HEALINGWOOD*¹* | Natural Oil |
| 46. | VIOLET LEAF ABS EGYPT LMR *¹* | Natural Oil |

| | | |
|---|---|---|
| *¹* Available from International Flavors & Fragrances Inc. | | |

**Table 2 - High-Impact Fragrance Ingredients**

| **No.** | **Trade Name** | **CAS** |
|---|---|---|
| 1. | ACETOPHENONE | 98-86-2 |
| 2. | AGRUNITRILE | 51566-62-2 |
| 3. | ALD AA TRIPLAL BHT | 68039-49-6 |
| 4. | ALD C-10 | 112-31-2 |
| 5. | ALD C-11 MOA BHT | 19009-56-4 |
| 6. | ALD C-11 ULENIC TOCO | 112-45-8 |
| 7. | ALD C-11 UNDECYLIC TOCO | 112-44-7 |
| 8. | ALD C-12 LAURIC TOCO | 112-54-9 |
| 9. | ALD C-12 MNA TOCO | 110-41-8 |
| 10. | ALD C-16 STRAWB#2 | 77-83-8 |
| 11. | ALD C-18 | 104-61-0 |
| 12. | ALD C-8 TOCO | 124-13-0 |
| 13. | ALD C-9 TOCO | 124-19-6 |
| 14. | AMBERTONIC | 1392325-86-8 |
| 15. | AMBRETTOLIDE | 28645-51-4 |
| 16. | AQUAFLORA TOCO | 1339119-15-1 |
| 17. | BENZALD FFC | 100-52-7 |
| 18. | CASHMERAN | 33704-61-9 |
| 19. | CEDRAMBER | 67874-81-1 |
| 20. | CITRAL NEW | 5392-40-5 |
| 21. | COOLWOOD | 1340502-69-3 |
| 22. | CRESOL PARA EXTRA | 106-44-5 |
| 23. | CRISTALFIZZ | 1093653-57-6 |
| 24. | CYCLAPROP | 68912-13-0 |
| 25. | CYCLEMAX | 7775-00-0 |
| 26. | DAMASCENONE TOCO | 23696-85-7 |
| 27. | DAMASCONE DELTA | 71048-82-3 |
| 28. | DELPHONE | 4819-67-4 |
| 29. | DIPHEN OXIDE | 101-84-8 |
| 30. | DORIFFOX | 149713-23-5 |
| 31. | ETH ACETO ACET | 141-97-9 |
| 32. | ETH CAPROATE | 123-66-0 |
| 33. | ETH PHEN GLYC | 121-39-1 |
| 34. | ETH SAL | 118-61-6 |
| 35. | ETH-2-METH BUTY | 7452-79-1 |
| 36. | EUCALYPTOL USP | 470-82-6 |
| 37. | FLORHYDRAL TOCO (ELINCS) | 125109-85-5 |
| 38. | FRUITATE (ELINCS) | 129520-41-8 |
| 39. | HEXADECANOLIDE BHT | 109-29-5 |
| 40. | HEXENYL ISOBUTY,CIS-3 (VERDURAL B) | 41519-23-7 |
| 41. | INDOLE | 120-72-9 |
| 42. | IONONE ALPHA TOCO | 127-41-3 |
| 43. | IRONE V BHT | 79-69-6 |
| 44. | ISO CYCLO CITRAL TOCO | 1423-46-7 |
| 45. | LACTONE OF CIS JASMONE TOCO | 70851-61-5 |
| 4 6. | LEMONILE | 61792-11-8 |
| 47. | MELONAL TOCO | 106-72-9 |
| 48. | MENTHONE 85 | 89-80-5 |
| 49. | METH BENZOATE | 93-58-3 |
| 50. | METH CINNAMATE TOCO | 103-26-4 |
| 51. | METH DH JASMONATE | 24851-98-7 |
| 52. | METH HEPTYL KETONE | 821-55-6 |
| 53. | METH JASMONATE TOCO | 39924-52-2 |
| 54. | METH OCTIN CARBONATE | 111-80-8 |
| 55. | METH PARA CRESOL | 104-93-8 |
| 56. | METH TUBERATE RD | 35205-76-6 |
| 57. | MUSCEMOR (ELINCS) | 82356-51-2 |
| 58. | MYRAC ALD BHT | 52475-89-5 |
| 59. | NECTARYL LRG 1371 ELINCS | 95962-14-4 |
| 60. | OCEANOL | 33662-58-7 |
| 61. | OPERANIDE (ELINCS) | 823178-41-2 |
| 62. | PHEN ETH ACET | 103-45-7 |
| 63. | PINEAPPLE CMPD | 3658-77-3 |
| 64. | PINO ACETALD TOCO | 33885-51-7 |
| 65. | ROSALVA | 13019-22-2 |
| 66. | ROSE OXIDE TOCO | 16409-43-1 |
| 67. | ROSETHYL | 64988-06-3 |
| 68. | SAFRALEINE | 54440-17-4 |
| 69. | SINFONIDE | 1315251-11-6 |
| 70. | STARFLEUR TOCO | 1254940-85-6 |
| 71. | SYLVONIC | 98-55-5 |
| 72. | TERPINOLENE P UB BHT | 586-62-9 |
| 73. | TOFFEE LACTONE 2067 | 705-86-2 706-14-9 |
| 74. | TOFFEETONE FOR NON TSCA USE ONLY | 13537-82-1 |
| 75. | TONKALACTONE | 54491-17-7 |
| 76. | TRIFERNAL BHT | 16251-77-7 |
| 77. | ULTRAVANIL Q COLIPA | 2563-07-7 |
| 78. | UNDECALACTONE,DEL T A | 710-04-3 |
| 79. | UNDECAVERTOL TOCO | 81782-77-6 |
| 80. | V ANITROPE | 94-86-0 |
| 81. | VARAMOL-106 | 7786-61-0 |
| 82. | VELTOL PLUS | 4940-11-8 |
| 83. | VERAMOSS | 4707-47-5 |
| 84. | VERIDIAN | 811412-48-3 |
| 85. | VERTOLIFF TOCO | 36635-35-5 |
| 86. | VERTONIC | 1945993-03-2 |
| 87. | VETIVER ACET HAITI TOCO BLO | 84082-84-8 |
| 88. | YARA YARA | 93-04-9 |
| 89. | YLANGANATE | 89-71-4 |
| 90. | ARMOISE OIL PURE*¹* | Natural oil |
| 91. | BLKCURRANT BUD ABS BURGUNDY LMR FOR LIFE *¹* | Natural oil |
| 92. | CHAMOMILE OIL ENG *³* | Natural oil |
| 93. | CHAMOMILE OIL ROMAN LMR SFO *¹* | Natural oil |
| 94. | CINNAMON BARK ESSENTIAL LMR *¹* | Natural oil |
| 95. | DAVANA OIL LMR FLG SFO *¹* | Natural oil |
| 96. | EUGENOL NAT EX CLOVE LEAF OIL | Natural oil |
| 97. | JASMIN ABS EGYPT LMR *¹* | Natural oil |
| 98. | ORANGE FLOWER WATER ABS TUNISIA LMR *¹* | Natural oil |
| 99. | OSMANTHUS ABS LMR *¹* | Natural oil |
| 100. | PATCHOULI OIL LIGHT BLO ² | Natural oil |
| 101. | PEPPER PINK CO2 LMR *¹* | Natural oil |
| 102. | ROSE ABS DAMASCENA PURE BLO *¹* | Natural oil |
| 103. | SINENSAL NATURAL 20 EX ORANGE *³* | Natural oil |
| 104. | THYME OIL WHITE SPAIN BLO *¹* | Natural oil |

| | | |
|---|---|---|
| *¹* Available from International Flavors & Fragrances Inc (New York). *²* Available from TRIPPER PTE Ltd. (Indonesia). *³* Available from TREATT & CO. Ltd. (United Kingdom). | | |

In addition to the fragrance ingredients listed in Tables 1 and 2, the fragrance may include at least one additional fragrance ingredient. Preferably, the fragrance may include 1, 2, 3, 4, 5, 6, 7, 8, 9, 10, 15, 20, 25, 30, 35, 40 or more additional fragrance ingredients, which are not listed in Tables 1 and 2. Non-limiting examples of such additional fragrance ingredients include those described in US 2018/0325786 A1, US 4,534,891, US 5,112,688, and US 5,145,842. Other suitable active materials that can be encapsulated include those listed in WO 2016/049456, pages 38-50.

The additional fragrance ingredients, when combined with one or more fragrance ingredients of Tables 1 and 2, constitute the total fragrance composition. In this respect, the balance of the 100 wt% relative to the total weight of the fragrance component is made up of one or more Ultra High-Impact and High-Impact fragrance ingredients of Tables 1 and 2 and one or more additional fragrance ingredients.

It has been surprisingly discovered that a fragrance that includes certain types of Ultra High-Impact and High-Impact fragrance ingredients results in High Performance fragrance ingredients for use in fabric care composition, preferably fabric conditioner, that improves fragrance profile and/or performance of the system. As used herein, the term "fragrance profile" means the description of how the fragrance is perceived by the human nose at any moment in time. The fragrance profile may change over time. It is a result of the combination of the base, heart and top notes, if present, of a fragrance. Base notes are characterized by providing animalic, woody, sweet, amber or musky characters, and not being very volatile. Heart notes are associated with desirable characters such as floral characters (*e*.*g*., jasmin, rose), fruity, marine, aromatic or spicy characters. The "top or head notes" provide citrusy, green, light, or fresh characters and tend to evaporate quickly due to their high volatility. A fragrance profile is composed of 2 characteristics: 'intensity' and 'character'. The 'intensity' relates to the perceived strength whilst 'character' refers to the odor impression or quality of the perfume, *i*.*e*., fresh, clean, *etc.*

The active material component (*e*.*g*., fragrance) of the core-shell microcapsules is preferably present at between 5.0% to 90.0%, preferably between 10.0% and 40.0% by weight of the microcapsule. When the fragrance includes a combination of the Ultra High-Impact fragrance ingredients and the High-Impact fragrance ingredients listed in Tables 1 and 2, the fragrance can be used in an aqueous fabric conditioner product at a significantly reduced dosage (*e*.*g*., at least 2-, 5- to 10-fold lower levels) as compared to a standard fragrance that does not include the Ultra High-Impact fragrance ingredients and High-Impact fragrance ingredients listed in Tables 1 and 2.

In particular, the fragrance of this invention can be used at a dosage level of ≤ 1 wt% relative to the total weight of the microcapsule composition without significantly impacting the fragrance profile, *i*.*e*., perceived fragrance intensity, perceived fragrance longevity and/or perceived fragrance fidelity, particularly for select characters (*e*.*g*., fresh and/or clean). In some aspects, the fragrance is used at a dosage level of less than or equal to 1 wt%, 0.99 wt%, 0.95 wt%, 0.9 wt%, 0.8 wt%, 0.7 wt%, 0.6 wt%, 0.5 wt%, 0.4 wt%, 0.3 wt%, 0.2 wt%, 0.1 wt%, 0.05 wt% or 0.01 wt% of the total weight of the aqueous fabric conditioner composition, or any range delimited by any pair of the foregoing values.

In addition to the active materials, the present invention also contemplates the incorporation of additional components including core modifier materials in the core encapsulated by the microcapsule wall. Other components include solubility modifiers, density modifiers, stabilizers, viscosity modifiers, pH modifiers, deposition aids, capsule formation aids, catalysts, processing aids or any combination thereof. These components can be present in the wall or core of the microcapsules, or outside the microcapsules in the microcapsule composition to improve solubility, stability, deposition, capsule formation, and the like. Further, the additional components may be added after and/or during the preparation of the microcapsule composition of this invention.

Prepolymers prepared in accordance with the methods described herein are suitable for many applications including microcapsules, nanocapsules, laminated pouches, and the like. In certain aspects, the prepolymers herein are used in the preparation of microcapsules. Accordingly, this invention provides for biodegradable core-shell microcapsules including an isocyanate-biodegradable polymer shell composed of the reaction product of a biodegradable, isocyanate-terminated prepolymer with a crosslinker and optionally a polyelectrolyte emulsifier under aqueous conditions; and a core comprising an active material. The biodegradable core-shell microcapsules are prepared by (a) reacting, under anhydrous conditions, a polyisocyanate dissolved in a solution with a biodegradable polymer in the presence of a catalyst to form an isocyanate-terminated prepolymer; (b) emulsifying the isocyanate-terminated prepolymer with an aqueous solution to form an emulsion; and (c) crosslinking the isocyanate-terminated prepolymer to form biodegradable core-shell microcapsules. In particular aspects, the polyisocyanate is dissolved in a solution that is (i) a water-soluble solvent, (ii) a water-soluble solvent including an active material or a portion thereof, or (iii) an active material. Preferable, the active material is a fragrance, flavor, agricultural active, pharmaceutical active, nutraceutical active, animal nutrition active, food active, microbio active, malodor counteractant, and/or cosmetic active, more preferably a fragrance.

In some aspects, the biodegradable polymer has the capacity to function as an emulsifier. However, in instances where the biodegradable polymer is not an emulsifier, the aqueous solution may optionally include an emulsifier, preferably a polyelectrolyte emulsifier that is soluble or dispersible in the aqueous solution. Like the biodegradable polymer and prepolymer, the polyelectrolyte emulsifier is ideally biodegradable, *i*.*e*., at least 60% biodegradable in 60 days according to OECD testing. Examples of polyelectrolyte emulsifier include, but are not limited to, gelatin, collagen, chitosan, modified guar, modified glucan, gum Arabic (gum acacia), modified gum Arabic (modified gum acacia), proteins, hydrolyzed proteins, fermented proteins, hydrophobin, enzymes, partially neutralized citric acid ester, alginate, carrageenan, pectin, modified starch, modified cellulose, or a combination thereof. In particular aspects, the polyelectrolyte emulsifier is gelatin, chitosan, modified guar, modified glucan, gum Arabic or a combination thereof.

To achieve the desired performance characteristics (*i*.*e*., active material retention and controlled release), the prepolymer is crosslinked with one or more crosslinking agents thereby forming the microcapsule wall. As used herein, a "crosslink" is a bond, atom, or group linking the chains of atoms in a prepolymer. In accordance with certain aspects of this invention, the prepolymer is crosslinked with an oxidized sugar. An "oxidized sugar" refers to monosaccharides, oligosaccharides, or polysaccharides that have been treated with an oxidizing agent or oxidant to generate reactive aldehyde groups. Ideally, the sugar has a molecular weight of less than 1000 g/mol and is soluble or dispersible in the aqueous phase. Examples of sugars of use in this invention include, but are not limited to, glucose, glucosamine, sucrose, maltose, lactose, maltodextrin, cyclodextrin, hydrolyzed polysaccharides, or any combination thereof. Preferably, the sugar to be oxidized is sucrose, glucosamine, maltodextrin or cyclodextrin, or more preferably sucrose. Suitable oxidants for obtaining oxidized sugars include, but are not limited to, sodium periodate, hydrogen peroxide, a laccase, or an oxidase, preferably sodium periodate. Use of oxidized sugars as crosslinkers has been found to highly crosslink the prepolymer and polyelectrolyte emulsifier. In particular, the average molecular weight of crosslinked prepolymer and polyelectrolyte emulsifier is at least 2, at least 3, at least 4, at least 5, at least 6, at least 7, at least 8, at least 9 or at least 10, preferably at least 2, at least 4, or at least 10, of the average molecular weight of non-crosslinked prepolymer and polyelectrolyte emulsifier, as determined by size exclusion chromatography.

Subsequent to the addition of a crosslinker, the method may further include a step of curing the microcapsules. As used herein, "curing" refers to a toughening or hardening process of a polymer brought about by heat, chemical additives, or light radiation. In some aspects, the microcapsules are cured at elevated temperatures, *e*.*g*., between 25°C and 250°C, preferably between 40°C and 85°C, for about 30 minutes to 24 hours, preferably 1 hour to 4 hours.

The method may alternatively or further include a step of drying the microcapsules to remove water. The drying temperature is in the range of 20°C and 250°C, or at room temperature. In some aspects, the microcapsules are dried by a dehumidifier configured to supply desiccated air to the microcapsules, a radiant heat source for facilitating drying of the microcapsules or submitting the microcapsules under a gas flow to obtain dried free-flowing microcapsules. It is understood that any standard method known by a person skilled in the art to perform such drying is also applicable.

Compared to conventional polyisocyanate and biodegradable polymer microcapsules, which are formed by self-condensation of polyisocyanate and a separate layer of biopolymer(s), the wall of the present microcapsules is formed from a prepolymer, thereby substantively or completely eliminating self-condensation of polyisocyanate. Preferably, the biodegradable core-shell microcapsules of this invention are substantively or completely free of self-condensation of polyisocyanate and therefore exhibit superior biodegradability properties (*i*.*e*., by avoiding and/or reducing blends of biodegradable and non-biodegradable materials). In particular, the obtained slurry of biodegradable core-shell microcapsules has a level of self-condensation of polyisocyanate (*i*.*e*., polyurea and/or polyurethane) that is below 10%, below 5%, below 3%, below 1% or below 0.5%, relative to total weight of polyisocyanate used to form wall of the microcapsules.

Preferably, the materials used to form the microcapsules of the present invention do not form a blend of biodegradable materials and non-biodegradable materials. In other aspects, if the materials used to form the microcapsule walls do form a blend, then the level of the resultant blend is not significant (*i.e*., ≤ 10%, ≤ 5%, ≤ 3%, ≤ 1% or ≤ 0.5% of non-biodegradable materials). In further aspects, if the materials used to form the microcapsule (or the microcapsule walls) do form a blend, then the biodegradation rate of all components of the blend is at least 20%, 30%, 40%, 50%, 60%, 65%, 70%, 75%, 80%, 85%, 90%, 95% or 98%, within 60 days according to OECD301F or OECD310.

Moreover, in conventional polyisocyanate and biodegradable polymer microcapsules, neither the biodegradable polymer nor any emulsifier is crosslinked. By comparison the biodegradable polymer and emulsifier (if included) are both highly crosslinked in the present microcapsules. In this respect, whereas the biodegradable polymer and emulsifier in conventional microcapsules can be washed away upon a change in pH, the microcapsule wall of this invention is pH stable, over a wide pH range, for example of 2-11.

Using the method of this invention, a relative high encapsulation efficiency is achieved. "Encapsulation efficiency" or "microencapsulation efficiency" represents the proportion of the active material core that is not available to an extracting solvent under specified test conditions. In accordance with the method of this invention, microencapsulation efficiencies in the range of 50% to 99.9% are attainable, or more preferably 60% to 99.7%. In particular, encapsulation efficiencies of at least 90%, 92%, 94%, 96%, 98%, or 99% are achieved.

In some aspects, the core-shell microcapsule is composed of between 0.1% and 20%, preferably between 5% and 10%, most preferably 7% by weight prepolymer; between 0.1% and 20%, preferably between 5% and 10%, most preferably 7% by weight polyelectrolyte emulsifier; between 1% and 80%, preferably between 50% and 70%, most preferably 66% by weight active material; between 1% and 70%, preferably between 20% and 40%, most preferably 27% by weight solvent; and between 0.1% and 10%, preferably between 3% and 6%, most preferably 4% by weight crosslinker, wherein the weight is relative to the total weight of the microcapsule.

In other aspects, a slurry including the core-shell microcapsules of this invention is composed of between 0.25% and 10% by weight biodegradable polymer, between 0.01% and 2% by weight polyisocyanate, between 0.001% and 1% by weight catalyst, between 0.1% and 10% by weight polyelectrolyte emulsifier, between 0.01% and 50% by weight active material, between 0.01% and 40% by weight solvent, between 0.01% and 5% by weight crosslinker, with the remaining percentage being water, and wherein the weight is relative to the total weight of the microcapsule.

The microcapsules of this invention each have a particle size (in diameter) in the range of 0.1 micron to 1000 microns (*e*.*g*., 0.5 micron to 500 microns, 1 micron to 200 microns, 1 micron to 100 microns, and 1 micron to 50 micron) with a lower limit of 0.1 micron, 0.5 micron, 1 micron, 2 microns, 5 microns and 20 microns, and an upper limit of 1000 microns, 500 microns, 200 microns, 100 microns, 75 microns, 50 microns, 30 microns, 20 microns, 10 microns and 5 microns.

Another aspect of the present invention is the desire to move towards the use of fragrance ingredients and/or microcapsules derived from "Green Chemistry" principles. Green Chemistry is focused on the design of products and processes that minimize environmental impact, particularly by using renewable feedstocks. In other words, the raw material or feedstock used to make the fragrance ingredients and/or microcapsules should be sustainable rather than depleting whenever technically and economically practicable. Preferably, the fragrance component and/or microcapsules of the present invention has a bio-renewable carbon (BRC) content of at least 50%, at least 55%, at least 60%, at least 65%, at least 70%, at least 75%, at least 80%, at least 85%, at least 90% or at least 95%. As used herein, "BRC" refers to carbon that is part of the earth's natural environment and non-fossil-based carbon. BRC are naturally occurring renewable, repurposed and/or upcycled carbon resources that can be replenished to replace the portion depleted by usage and consumption, either through natural reproduction, or other recurring processes in a finite amount of time (such as within a human lifetime). BRC would exclude carbon that comes from virgin crude oil.

In some aspects, one or more non-confined or unencapsulated active materials can also be included post-curing. Such active materials may be the same or different than the encapsulated active material and may be included at a level of from 0.01% to 20%, or more preferably from 2% to 10% by weight of the microcapsule composition (*i*.*e*., microcapsule slurry).

The microcapsules of this invention can also be combined with one or more other delivery systems such as polymer-assisted delivery compositions (see US 8,187,580), fiber-assisted delivery compositions (US 2010/0305021), cyclodextrin host-guest complexes (US 6,287,603 and US 2002/0019369), pro-fragrances (WO 2000/072816 and EP 0 922 084), and any combination thereof. More exemplary delivery systems that can be incorporated are coacervate capsules, cyclodextrin delivery systems, and pro-perfumes.

Furthermore, microcapsules having one or more different characteristics can be combined to provide desirable or tailored release profiles and/or stability. In particular, the microcapsule composition can include a combination of two or more types of microcapsules that differ in their encapsulating wall materials, microcapsule size, amounts of wall materials, the thickness of the wall, the degree of polymerization, the degree of crosslinking, ratios between the wall materials and the active material, core modifiers, scavengers, active materials, cure temperatures, heating rates during the curing, curing times, the rupture force or fracture strength, or a combination thereof. In some aspects, the microcapsule composition is composed of two, three, four, five, six, seven or more different types of capsules that differ by one or more of the above-referenced characteristics.

When assessing storage stability, fragrance retention within the microcapsule may be measured directly after storage, at a desired temperature and time periods such as four weeks, six weeks, two months, three months or more in a consumer product base. The preferred manner is to measure total headspace of the consumer product at the specified time and to compare the results to the headspace of a control consumer product made to represent 0% retention via direct addition of the total amount of fragrance present. Alternatively, the consumer product may be performance tested after the storage period and the performance compared to the fresh product, either analytically or by sensory evaluation. This measurement often involves either measuring the fragrance headspace over a substrate used with the product, or odor evaluation of the same substrate.

In certain aspects, retention of the active material in the core of the instant microcapsules is assessed in a consumer product base, *e*.*g*., under storage conditions such as at a temperature in the range of 25°C to 40°C, or more preferably in the range of 30°C to 37°C, or most preferably 37°C, for an extended period of time of at least 2 weeks, 4 weeks, 6 weeks, 8 weeks, 16 weeks, or 32 weeks. In certain aspects, the microcapsules of this invention retain at least 30%, 40%, 45%, 50%, 55%, 60%, 65%, 70%, 75%, 80%, 85%, 90%, 95%, or 99% of the active material when added to a consumer product base. In particular aspects, the microcapsules of this invention, when added to a consumer product base, retain between 40% and 90% of the active material after being stored at 37°C for at least 4 weeks, 8 weeks or 12 weeks. Alternatively stated, the microcapsules of this invention lose less than 50% of the active material due to leakage when added to a consumer product base and stored for 8 weeks at 37°C.

The biodegradable core-shell microcapsules of this invention are well-suited for inclusion in any of a variety of consumer products where controlled release of active materials (*e*.*g*., fragrances or flavors) is desired. The microcapsules of this invention can be added to a consumer product base directly or be printed onto a product base or a movable product conveyor (*e*.*g*., a non-stick belt) for drying. See WO 2019/212896 A1. The biodegradable core-shell microcapsules can be added to the consumer product base at a level in the range of 0.001% to 50%, or more preferably 0.01% to 50% by weight of the consumer product base. In particular, the biodegradable core-shell microcapsules are suitably included in a consumer product such as a pharmaceutical, agricultural or cosmetic formulation. Examples of consumer products include, but are not limited to, a fabric softener, fabric conditioner, detergent, scent booster, fabric refresher spray, body wash, body soap, shampoo, hair conditioner, body spray, hair refresher spray, hair dye, hair moisturizer, skin moisturizer, hair treatment, antiperspirant, deodorant, skin treatment, insect repellant, candle, surface cleaner, bathroom cleaner, bleach, cat litter, refresher spray or pesticide.

The values and dimensions disclosed herein are not to be understood as being strictly limited to the exact numerical values recited. Instead, unless otherwise specified, each such value is intended to mean both the recited value and a functionally equivalent range surrounding that value. For example, a value disclosed as "50%" is intended to mean "about 50%." Moreover, ranges include those provided as well as any range delimited by any pair of the recited values. For example, a range disclosed as 0.5% to 5.0% encompasses a range of 0.5% to 1.0%, 0.8% to 3.0%, 1.0% to 5.0%, etc.

### EXAMPLES

The following non-limiting examples are provided to further illustrate the present invention and are not to be construed as limitations of the invention, as many variations of the present invention are possible without departing from its spirit or scope.

### Example 1: Gelatin Prepolymer Microcapsules

The oil phase was prepared separately by mixing Magnitude (a fragrance commercially available from International Flavors & Fragrances Inc., NY), caprylic/capric triglyceride (commercially available under the tradename NEOBEE^{®} oil M-5, Stepan, Chicago, IL) and polyisocyanate (commercially available under the tradename TAKENATE^{®} D-110N, Mitsui Chemicals, Japan). Gelatin was subsequently dispersed in the oil phase under constant mixing. The mixture was heated with constant mixing and 1,4-diazabicyclo[2.2.2]octane (DABCO) was optionally added to catalyze prepolymer formation for the times and temperatures indicated in Tables 3-6. Separately, an aqueous solution of gum Arabic was prepared and then emulsified with the oil phase at 6000 rpm for 3 minutes. After allowing the mixture to stir at 40°C for 1 hour, the pH was gradually adjusted to 3.5 using 50% citric acid. A crosslinking solution mixture of sucrose and sodium periodate was prepared and mixed at room temperature for 30 minutes. The crosslinking solution was then added to the emulsion and stirring was maintained at 40°C for 4 hours. The resulting microcapsules were designated Microcapsules 1-14.

**TABLE 3**

| **Microcapsule Component** | **Microcapsule (%)*¹*** | | |
|---|---|---|---|
| | **1** | **2** | **3** |
| Water | 82% | 82% | 82% |
| Gum Arabic | 2.0% | 2.0% | 2.0% |
| Gelatin | 1.5% | 1.5% | 1.5% |
| Fragrance | 10% | 10% | 10% |
| NEOBEE^{®} | 2.5% | 2.5% | 2.5% |
| TAKENATE^{®} | 0.25% | 0.25% | 0.25% |
| Citric Acid | 0.30% | 0.30% | 0.30% |
| Sucrose | 1.0% | 1.0% | 1.0% |
| Sodium Periodate | 0.10% | 0.10% | 0.10% |
| DABCO | 0.015% | 0.015% | 0.015% |
| **Microcapsule Condition** | | | |
| Temperature (°C) | 60°C | 60°C | 60°C |
| Reaction Time (minutes) | 10 mins | 30 mins | 60 mins |

| | | | |
|---|---|---|---|
| *¹* Percentages are based on total weight of the Microcapsule. | | | |

**TABLE 4**

| **Microcapsule Component** | **Microcapsule (%)*¹*** | | |
|---|---|---|---|
| | **4** | **5** | **6** |
| Water | 82% | 82% | 82% |
| Gum Arabic | 2.0% | 2.0% | 2.0% |
| Gelatin | 1.5% | 1.5% | 1.5% |
| Fragrance | 10% | 10% | 10% |
| NEOBEE^{®} | 2.5% | 2.5% | 2.5% |
| TAKENATE^{®} | 0.25% | 0.25% | 0.25% |
| Citric Acid | 0.30% | 0.30% | 0.30% |
| Sucrose | 1.0% | 1.0% | 1.0% |
| Sodium Periodate | 0.10% | 0.10% | 0.10% |
| DABCO | 0.015% | 0.015% | 0.015% |
| **Microcapsule Condition** | | | |
| Temperature (°C) | RT*²* | RT*²* | RT*²* |
| Reaction Time (minutes) | 10 mins | 30 mins | 60 mins |

| | | | |
|---|---|---|---|
| *¹* Percentages are based on total weight of the Microcapsule. ² RT, room temperature. | | | |

**TABLE 5**

| **Microcapsule Component** | **Microcapsule (%)*¹*** | | | |
|---|---|---|---|---|
| | **7** | **8** | **9** | **10** |
| Water | 82% | 82% | 82% | 82% |
| Gum Arabic | 2.0% | 2.0% | 2.0% | 2.0% |
| Gelatin | 1.5% | 1.5% | 1.5% | 1.5% |
| Fragrance | 10% | 10% | 10% | 10% |
| NEOBEE^{®} | 2.5% | 2.5% | 2.5% | 2.5% |
| TAKENATE^{®} | 0.25% | 0.25% | 0.25% | 0.25% |
| Citric Acid | 0.30% | 0.30% | 0.30% | 0.30% |
| Sucrose | 1.0% | 1.0% | 1.0% | 1.0% |
| Sodium Periodate | 0.10% | 0.10% | 0.10% | 0.10% |
| DABCO | 0.10% | 0.10% | 0.10% | 0.10% |

| **Microcapsule Condition** | | | | |
|---|---|---|---|---|
| Temperature (°C) | 60°C | 60°C | RT | RT |
| Reaction Time (minutes) | 10 mins | 60 mins | 10 mins | 60 mins |

| | | | | |
|---|---|---|---|---|
| *¹* Percentages are based on total weight of the Microcapsule. ² RT, room temperature. | | | | |

**TABLE 6**

| **Microcapsule Component** | **Microcapsule (%)*¹*** | | | |
|---|---|---|---|---|
| | **11** | **12** | **13** | **14** |
| Water | 82% | 82% | 82% | 82% |
| Gum Arabic | 2.0% | 2.0% | 2.0% | 2.0% |
| Gelatin | 1.5% | 1.5% | 1.5% | 1.5% |
| Fragrance | 10% | 10% | 10% | 10% |
| NEOBEE^{®} | 2.5% | 2.5% | 2.5% | 2.5% |
| TAKENATE^{®} | 0.25% | 0.25% | 0.25% | 0.25% |
| Citric Acid | 0.30% | 0.30% | 0.30% | 0.30% |
| Sucrose | 1.0% | 1.0% | 1.0% | 1.0% |
| Sodium Periodate | 0.10% | 0.10% | 0.10% | 0.10% |
| DABCO | 0.00% | 0.00% | 0.00% | 0.00% |

| **Microcapsule Condition** | | | | |
|---|---|---|---|---|
| Temperature (°C) | 60°C | 60°C | RT*²* | RT*²* |
| Reaction Time (minutes) | 10 mins | 60 mins | 10 mins | 60 mins |

| | | | | |
|---|---|---|---|---|
| *¹* Percentages are based on total weight of the Microcapsule. ² RT, room temperature. | | | | |

The encapsulation efficiency (EE) of Microcapsule slurries 1-13 was obtained and is presented in Table 7.

**TABLE 7**

| **Microcapsule** | **EE** |
|---|---|
| 1 | 99% |
| 2 | 99% |
| 3 | 86% |
| 4 | 99% |
| 5 | 99% |
| 6 | 99% |
| 7 | 75% |
| 8 | 18% |
| 9 | 99% |
| 10 | 20% |
| 11 | 99% |
| 12 | 99% |
| 13 | 99% |
| 14 | 99% |

### Example 2: Reactivity of Gelatin and Polyisocyanate in Oil Phase

The oil phase was prepared separately by mixing benzyl benzoate and polyisocyanate (commercially available under the tradename TAKENATE^{®} D-110N, Mitsui Chemicals, Japan). Gelatin was subsequently dispersed in the oil phase under constant mixing. The mixture was heated with constant mixing and DABCO was optionally added to catalyze prepolymer formation for the times and temperatures indicated in Tables 3-6. As described in Example 1, microcapsules were formed and designated Microcapsules 1A-14A. Three additional samples were prepared under 1A-3A conditions; however, gelatin was omitted to demonstrate reactivity predominantly in the presence of gelatin. These microcapsules were designated 1A No Gelatin, 2A No Gelatin, and 3A No Gelatin. A 50 mg aliquot of each microcapsule was quenched with 7.9 g of MeOH. These samples were then analyzed for methyl urethane adduct by liquid chromatography (LC) in ppm. Polyisocyanate reactivity of Microcapsules 1A-14A and 1A-3A is presented in Table 8.

**TABLE 8**

| **Microcapsule** | **Polyisocyanate Reactivity** |
|---|---|
| 1A | 35% |
| 2A | 58% |
| 3A | 66% |
| 1A No Gelatin | 0% |
| 2A No Gelatin | 0% |
| 3A No Gelatin | 16% |
| 4 | 10% |
| 5 | 19% |
| 6 | 27% |
| 7 | 86% |
| 8 | 100% |
| 9 | 28% |
| 10 | 60% |
| 11 | 0% |
| 12 | 17% |
| 13 | 0% |
| 14 | 1% |

Based upon the results presented in Table 6 and Table 7, EE and polyisocyanate reactivity are influenced by reaction time, reaction temperature and/or catalyst concentrations. DABCO is essential to the formation of the prepolymer. Furthermore, the lack of reactivity without gelatin, validates the formation of prepolymer.

### Example 3: Gelatin Comparative Microcapsule 15

In this example, microcapsules were prepared as described in Example 1, except the biodegradable polymer was dispersed in the aqueous phase. The oil phase was prepared separately by mixing Magnitude (a fragrance commercially available from International Flavors & Fragrances Inc., NY), caprylic/capric triglyceride (sold under the tradename NEOBEE^{®} M-5), and polyisocyanate (sold under the tradename TAKENATE^{®} D-110N). Separately, a solution containing gum Arabic and gelatin was prepared at 60°C and subsequently emulsified with the oil phase at 6000 rpm for 3 minutes. After allowing the mixture to stir at 40°C for 1 hour, the pH was gradually adjusted to 3.5 using 50% citric acid.

### Example 4: Gelatin Comparative Microcapsule 16

In this example, microcapsules were prepared as described in Example 1, except the biodegradable polymer was dispersed in the aqueous phase and polyisocyanate was omitted. The oil phase was prepared separately by mixing Magnitude (a fragrance commercially available from International Flavors & Fragrances Inc., NY)with caprylic/capric triglyceride (sold under the tradename NEOBEE^{®} M-5). Separately, a solution containing gum Arabic and gelatin was prepared at 60°C and subsequently emulsified with the oil phase at 6000 rpm for 3 minutes. After allowing the mixture to stir at 40°C for 1 hour, the pH was gradually adjusted to 3.5 using 50% citric acid.

### Example 5: Microcapsule pH Stability

The pH of the microcapsules prepared in Example 1 (Microcapsule 1) and Example 3 (Comparative Microcapsule 15) was adjusted to pH 3.5, 5 and greater than 10 using 10% NaOH and microcapsule stability was monitored using a Motic BA310 microscope. The results of this analysis indicated that Microcapsule 1 was stable at pH 3.5, 5 and >10. In contrast, the microcapsule wall of comparative Microcapsule 15 dissolved at pH 5 and >10.

### Example 6: Validation of Prepolymer Formation

In this example, microcapsules were prepared as described in Example 1 (Microcapsule 1) with the following modifications. The oil phase was prepared by mixing benzyl benzoate and polyisocyanate (sold under the tradename TAKENATE^{®} D-110N). Gelatin was dispersed in the oil phase under constant mixing. The mixture was heated at constant mixing and DABCO was added to catalyze the prepolymer reaction for 10 minutes. The sample was then dissolved in dimethyl sulfoxide/lithium chloride solution and analyzed by multi-angle light scattering coupled with size exclusion chromatography (SEC-MALS) to determine the molar mass. For comparison, gelatin was also dissolved and analyzed under the same condition. The results of this analysis (Table 9) indicate an increase in molecular weight and size of gelatin by covalent modification with polyisocyanate.

**TABLE 9**

| **Sample** | **Mw (kDa)*¹*** | **Mz (kDa)*²*** | **PD (Mw/Mn)*³*** | **rz (nm)*⁴*** |
|---|---|---|---|---|
| Prepolymer | 359 | 1520 | 6.3 | 24 |
| Gelatin | 171 | 401 | 2.2 | 14 |

| | | | | |
|---|---|---|---|---|
| *¹* Mw, lower mass average molecular weight of the pre-polymer and gelatin. *²* Mz, higher mass average molecular weight of the pre-polymer and gelatin. *³* PD, polydispersity. *⁴* rz, z-average of root mean square radius of the prepolymer and gelatin. | | | | |

### Example 7: Validation of Highly Crosslinked Microcapsule Wall

Microcapsules prepared according to Example 1 (Microcapsule 1), Example 3 (Comparative Microcapsule 15), and Example 4 (Comparative Microcapsule 16) were washed 3 times with water to remove any residual unreacted materials. The material was then freeze dried to remove excess water and the fragrance extracted several times until analysis indicated that less than 3% of fragrance was detectable via standard gas chromatography (GC) analysis. Samples were then dried and analyzed (at the same weight percentage of dried material) using SEC-MALS to determine the molar mass and crosslinking. The results of this analysis (Table 10) demonstrated the significant increase in molecular weight (Mw, Mz) and size (rz) of the wall of Microcapsule 1 as compared to the molecular weight of the walls of Comparative Microcapsules 15 and 16.

PD refers to 'polydispersity' and characterizes the distribution of the molecular weights in a given polymer sample. PD represents the ratio of M_{w}/Mₙ which is the weight average molecular weight (M_{w}) divided by the number average molecular weight (Mₙ) of the polymer. When the PD is 1, the polymers in the sample are monodisperse (*i*.*e*., all polymers have consistent chain length, molecular weight and mass distribution). PD values greater than 1 mean that the polymers in the sample are polydisperse (*i*.*e*., the polymers have nonuniform chain length, molecular weight and mass distribution). The greater the PD value the more polydisperse are the polymers in the sample. The results of this analysis (Table 10) demonstrated the introduction of higher molecular weight polymers, therefore crosslinked on the microcapsule wall.

**TABLE 10**

| | **Mw (kDa)*¹*** | **Mz (kDa)*²*** | **PD (M_{w}/Mₙ)*³*** | **rz (nm)*⁴*** |
|---|---|---|---|---|
| Microcapsule 1 | 1218 | 6551 | 3.929 | 38.1 |
| Comparative Microcapsule 15 | 435 | 1075 | 2.332 | 15 |
| Comparative Microcapsule 16 | 419 | 1104 | 2.273 | 17 |

| | | | | |
|---|---|---|---|---|
| *¹* Mw, lower mass average molecular weight of the pre-polymer and gelatin. *²* Mz, higher mass average molecular weight of the pre-polymer and gelatin. *³* PD, polydispersity. *⁴* rz, z-average of root mean square radius of the prepolymer and gelatin. | | | | |

Light scattering chromatogram is a measure of concentration and mass of the particles. Fractions eluted at between 66 minutes and 86 minutes, which were signature peaks of gum Arabic and gelatin. Notably, microcapsule walls prepared according to Example 3 (Comparative Microcapsule 15) and Example 4 (Comparative Microcapsule 16) had minimal differences indicative of almost no crosslinking or increase in molar mass of either the gum Arabic or gelatin. By comparison, the microcapsule prepared according to Example 1 (Microcapsule 1) showed a significant increase in intensity indicating the increase in molar mass relative to the same weight% capsule wall concentration. Furthermore, peak shifts to the left, the elution time change, signified the increase in molar mass of the soluble portion for this analysis as reflected in Table 10. The resulting increase in molar mass and size of the wall material in Microcapsule 1 is reflective of a high degree of crosslinking of the prepolymer and emulsifier.

### Example 8: Biodegradation of Microcapsules

Example 1 (Microcapsule 1) and Examples 3 and 4 (Comparative Microcapsules 15 and 16) were washed as described herein above Example 7 and evaluated by OECD301F. Microcapsule 1, a capsule according to the present invention, was determined to have a biodegradation rate of 75% within 60 days. The biodegradation rate for Comparative Microcapsules 15 and 16 (not within the scope of the present invention) was also > 60% within 60 days (data not shown). It was also observed that Microcapsule 1 of the present invention can prevent the formation of a blend of biodegradable and non-biodegradable materials by analysis of capsule wall. In contrast, Comparative Microcapsule 15 was observed unable to prevent the formation of a blend of these materials. Comparative Microcapsule 16, which is just a gelatin coacervate without polyisocyanate, while it may be biodegradable, was observed to lack performance, stability and/or chemical cross-linking.

### Example 9: Chitosan Prepolymer Microcapsule 17

The oil phase was prepared separately by mixing Eden (a fragrance commercially available from International Flavors & Fragrances Inc., NY) at 10.0%, caprylic/capric triglyceride (sold under the tradename NEOBEE^{®} M-5) at 2.5% and polyisocyanate (sold under the tradename TAKENATE^{®} D-1 10N) at 0.25%. Chitosan (available from Glentham Life Sciences, Corsham, UK) at 1.5% was dispersed in the oil phase under constant mixing.

The mixture was heated to 60°C at constant mixing and DABCO at 0.015% was added to catalyze the reaction for 10 mins. Separately, an aqueous solution of gum Arabic at 2.0% was prepared together with 2.0% acetic acid. The aqueous solution and oil phase were mixed and emulsified at 6000 rpm for 3 minutes. The mixture was stirred at 40°C for 1 hour, then the pH was gradually adjusted to 5.5 using 5% sodium hydroxide. A crosslinking solution composed of sucrose at 1.0% and sodium periodate at 0.1% was prepared and mixed at room temperature for 30 minutes. The crosslinking solution was then added to the emulsion and the stirring was maintained at 40°C for 4 hours.

### Example 10: Cationic Guar Prepolymer Microcapsule 18

The oil phase was prepared separately by mixing Eden (a fragrance commercially available from International Flavors & Fragrances Inc., NY) at 10.0%, caprylic/capric triglyceride (sold under the tradename NEOBEE^{®} M-5) at 2.5% and polyisocyanate (sold under the tradename TAKENATE^{®} D-110N) at 0.25%. Cationic guar (available as N-HANCE^{™} C261N from Ashland Specialty Chemical, Delaware) at 1.5% was dispersed in the oil phase under constant mixing. The mixture was heated to 60°C at constant mixing and DABCO at 0.015% was added to catalyze the reaction for 10 mins. Separately, an aqueous solution of gum Arabic at 2.0% was prepared. The aqueous solution and oil phase were mixed and emulsified at 6000 rpm for 3 minutes. The mixture was stirred at 40°C for 1 hour, then the pH was gradually adjusted to 3.8 using 50% citric acid. A crosslinking solution composed of sucrose at 1.0% and sodium periodate at 0.1% was prepared and mixed at room temperature for 30 minutes. The crosslinking solution was then added to the emulsion and the stirring was maintained at 40°C for 4 hours.

### Example 11: Cationic Glucan Prepolymer Microcapsule 19

The oil phase was prepared separately by mixing Eden (a fragrance commercially available from International Flavors & Fragrances Inc., NY) at 10.0%, caprylic/capric triglyceride (sold under the tradename NEOBEE^{®} M-5) at 2.5% and polyisocyanate (sold under the tradename TAKENATE^{®} D-110N) at 0.25%. Cationic glucan having a degree of substitution per monomer glucan unit of less than 10% (a proprietary material to International Flavors & Fragrances Inc.) at 1.5% was dispersed in the oil phase under constant mixing. The mixture was heated to 60°C at constant mixing and DABCO at 0.015% was added to catalyze the reaction for 10 mins. Separately, an aqueous solution of gum Arabic at 2.0% was prepared. The aqueous solution and oil phase were mixed and emulsified at 6000 rpm for 3 minutes. The mixture was stirred at 40°C for 1 hour, then the pH was gradually adjusted to 2.8 using 50% citric acid. A crosslinking solution composed of sucrose at 1.0% and sodium periodate at 0.1% was prepared and mixed at room temperature for 30 minutes. The crosslinking solution was then added to the emulsion and the stirring was maintained at 40°C for 4 hours.

### Example 12: Collagen Prepolymer Microcapsule 20

The oil phase was prepared separately by mixing Eden (a fragrance commercially available from International Flavors & Fragrances Inc., NY) at 10.0%, caprylic/capric triglyceride (sold under the tradename NEOBEE^{®} M-5) at 2.5% and polyisocyanate (sold under the tradename TAKENATE^{®} D-110N) at 0.25%. Collagen (Biollagen^{™} available from JLand Biotech Co. Ltd., Jinjiang, China) at 1.5% was dispersed in the oil phase under constant mixing. The mixture was heated to 60°C at constant mixing and DABCO at 0.015% was added to catalyze the reaction for 10 mins. Separately, an aqueous solution of gum Arabic at 2.0% was prepared. The aqueous solution and oil phase were mixed and emulsified at 6000 rpm for 3 minutes. The mixture was stirred at 40°C for 1 hour, then the pH was gradually adjusted to 3.5 using 50% citric acid. A crosslinking solution composed of sucrose at 1.0% and sodium periodate at 0.1% was prepared and mixed at room temperature for 30 minutes. The crosslinking solution was then added to the emulsion and stirring was maintained at 40°C for 4 hours.

### Example 13: Gum Arabic Prepolymer Microcapsule 21

The oil phase was prepared separately by mixing Eden (a fragrance commercially available from International Flavors & Fragrances Inc., NY) at 10.0%, caprylic/capric triglyceride (sold under the tradename NEOBEE^{®} M-5) at 2.5% and polyisocyanate (sold under the tradename TAKENATE^{®} D-110N) at 0.25%. Gum Arabic at 2.0% was dispersed in the oil phase under constant mixing. The mixture was heated to 60°C at constant mixing and DABCO at 0.025% was added to catalyze the reaction for 10 mins. Separately, an aqueous solution of gelatin at 1.5% was prepared. The aqueous solution and oil phase were mixed and emulsified at 6000 rpm for 3 minutes. The mixture was stirred at 40°C for 1 hour, then the pH was gradually adjusted to 3.5 using 50% citric acid. A crosslinking solution composed of sucrose at 1.0% and sodium periodate at 0.1% was prepared and mixed at room temperature for 30 minutes. The crosslinking solution was then added to the emulsion and the stirring was maintained at 40°C for 4 hours.

### Example 14: Gelatin Prepolymer with Carboxymethyl Cellulose (Degree of Substitution of 0.4), Microcapsule 22

The oil phase was prepared separately by mixing Eden (a fragrance commercially available from International Flavors & Fragrances Inc., NY) at 10%, caprylic/capric triglyceride (sold under the tradename NEOBEE^{®} M-5) at 2.5%, and polyisocyanate (sold under the tradename TAKENATE^{®} D-110N) at 0.25%. Gelatin at 1.5% was dispersed in the oil phase under constant mixing. The mixture was heated to 60°C with constant mixing and DABCO was added to catalyze the reaction for 10 mins. Separately, an aqueous solution of 2.0% carboxymethyl cellulose (prepared according to EP 2552968 B1 with Degree of Substitution (DS) of 0.4) was prepared. The aqueous solution and oil phase were mixed and emulsified at 6000 rpm for 3 minutes. The mixture was stirred at 40°C for 1 hour, then the pH was gradually adjusted to 4.6 using 50% citric acid. A crosslinking solution composed of sucrose and sodium periodate was prepared and mixed at room temperature for 30 minutes. The crosslinking solution was then added to the emulsion and stirring was maintained at 40°C for 4 hours.

### Example 15: Gelatin Prepolymer with Carboxymethyl Cellulose (Degree of Substitution of 0.2), Microcapsule 23

The oil phase was prepared separately by mixing Eden (a fragrance commercially available from International Flavors & Fragrances Inc., NY) at 10%, caprylic/capric triglyceride (sold under the tradename NEOBEE^{®} M-5) at 2.5%, and polyisocyanate (sold under the tradename TAKENATE^{®} D-110N) at 0.25%. Gelatin (beef) at 1.5% was dispersed in the oil phase under constant mixing. The mixture was heated to 60°C with constant mixing and DABCO was added to catalyze the reaction for 10 mins. Separately, an aqueous solution of 2.0% carboxymethyl cellulose (prepared according to EP 2552968 B1 with DS of 0.2) was prepared. The aqueous solution and oil phase were mixed and emulsified at 6000 rpm for 3 minutes. The mixture was stirred at 40°C for 1 hour, then the pH was gradually adjusted to 3.5 using 50% citric acid. A crosslinking solution composed of sucrose and sodium periodate was prepared and mixed at room temperature for 30 minutes. The crosslinking solution was then added to the emulsion and stirring was maintained at 40°C for 4 hours.

### Example 16: Encapsulation Efficiencies of Microcapsules 17-23

The encapsulation efficiency (EE) of Microcapsules 17-23 was obtained and is presented in Table 11.

**TABLE 11**

| **Microcapsule** | **EE** |
|---|---|
| 17 | 99% |
| 18 | 99% |
| 19 | 97% |
| 20 | 99% |
| 21 | 99% |
| 22 | 98% |
| 23 | 99% |

The results of these analyses indicated that isocyanate-terminated prepolymers could be formed with gelatin (-NH₂), chitosan (-NH₂, -OH), modified guar (-OH), modified glucans (-OH), collagen (-NH₂, -OH), proteins (-NH₂, -OH), partially hydrolyzed citric acid esters (-COOH), gum Arabic (-OH, -COOH), alginate (-OH, -COOH) and pectin (-OH, -COOH). In this respect, a plurality of biodegradable polymers with -NH₂, -OH or - COOH functional groups can be used to prepare isocyanate-terminated prepolymers of use in microencapsulation according to the present invention.

### Example 17: Microcapsule Performance

To evaluate fragrance profile performance, microcapsule slurries (as described above) are blended into a model fabric conditioner base (19% active level) as shown in Table 12. The microcapsules encapsulated two different fragrances, Magnitude fragrance and Mermaid fragrance (fragrances commercially available from International Flavors & Fragrances Inc., NY).

**TABLE 12**

| **Material** | **Amount** (g) |
|---|---|
| REWOQUAT^{®} WE 18 (Esterquat; Evonik, 90% in IPA) | 23.46 |
| PROXEL^{®} GLX (1,2-benzisothiazolin-3-one) | 0.11 |
| Water | 76.1 |
| Calcium Chloride (25%) | 0.33 |

The fragrance load is 0.1% neat oil equivalent (NOE). The fragrance intensity of the perfumes encapsulated by the microcapsules is evaluated by conducting a laundry experiment using accepted experimental protocols using European wash machine (Miele). Terry towels are used for the washing experiments and are washed with European fabric conditioners containing fragrance loaded capsules. Washed samples are removed from the washing machine and line dried overnight. The samples are evaluated by a panel of 24 trained judges at three different stages and rated on a scale ranging from 0 to 30.

"Pre-rub" refers to the evaluations of the towels by panelists before the folding of the towels. "Gentle handling" refers to the folding of the towels twice, followed by the evaluation of the towels by the panelists. "Post-rub" refers to vigorous application of mechanical force using both hands to rub the cloths at least once to rupture the capsules and then evaluated for signs of released fragrance. A numerical value of 0 indicates that the fabric produced no signs of released fragrance, 5 indicates that the fabric only produced weak intensity and 30 indicates a very strong smell of released fragrance from the microcapsules.

The results indicated that biodegradable Microcapsule 1 prepared in accordance with the methods and biopolymers herein demonstrated intensity levels of 7.9 "Pre-rub" and 13.6 "Post-rub", respectively, showing superior performance as compared to neat fragrances below intensities of 4 for both "Pre-rub" and "Post-rub" (*i*.*e*., non-encapsulated).

### Example 18: Encapsulated High Performance Fragrance Compositions

High Performance fragrance Examples 1 and 2 are provided in Table 13 and represents formulation of fragrances comprising encapsulated High Performance fragrance ingredients according to the present invention. Comparative Fragrance Example 1 is also provided in Table 13, which represents encapsulated standard fragrance not intended to form the High Performance fragrance of the present invention. The following fragrance formulations are made by mixing the listed ingredients in the listed proportions in Table 13 at room temperature. The capsules used are Example 1 (Microcapsule 1) and Examples 3 and 4 (Comparative Microcapsules 15 and 16).

**TABLE 13**

| **Ingredient Name** | **CAS Number** | **Comparative Fragrance Example** 1 | **High Performance Fragrance Example 1** | **High Performance Fragrance Example 2** |
|---|---|---|---|---|
| ADOXAL TOCO | 141-13-9 | 14.29 | 35.00 | 0.00 |
| ALD AA TRIPLAL TOCO | 68039-49-6 | 28.57 | 60.00 | 65.00 |
| ALD C-10 TOCO | 112-31-2 | 0.00 | 0.00 | 75.00 |
| ALD C-12 MNA TOCO | 110-41-8 | 0.00 | 0.00 | 120.00 |
| ALLYL AMYL GLYCOLATE BHT | 67634-00-8 | 28.57 | 0.00 | 0.00 |
| AMBER XTREME TM | 476332-65-7, 647828-16-8 | 8.57 | 25.00 | 6.00 |
| AMBERTONIC | 1392325-86-8 | 0.00 | 0.00 | 15.00 |
| CASHMERAN | 33704-61-9 | 0.00 | 0.00 | 45.00 |
| CYCLACET | 2500-83-6 | 285.71 | 75.00 | 0.00 |
| CYCLAPROP | 68912-13-0 | 142.86 | 184.50 | 0.00 |
| CYCLOHEXYL SAL (ELINCS) | 25485-88-5 | 0.00 | 0.00 | 21.00 |
| DAMASCONE DELTA TOCO | 71048-82-3 | 0.00 | 0.00 | 50.00 |
| DIHYDRO TERPINEOL | 498-81-7 | 4.06 | 0.00 | 0.00 |
| DIHYDRO TERPINYL ACET | 58985-18-5 | 94.29 | 0.00 | 0.00 |
| EUCALYPTOL USP CSM | 470-82-6 | 0.00 | 0.00 | 100.00 |
| FRUCTALATE (ELINCS) | 72903-27-6 | 0.00 | 0.00 | 25.00 |
| GALBANUM OIL LMR *¹* | natural oil | 0.00 | 0.40 | 0.50 |
| GALBASCONE ALPHA 95 PRG TOCO | 56973-85-4 | 0.00 | 10.00 | 4.00 |
| GALBASCONE PRG TOCO | 56973-85-4, 56973-84-3 | 22.86 | 50.00 | 15.00 |
| GERANIOL 980 PURE | 106-24-1 | 0.00 | 0.00 | 15.00 |
| HERBALIME | 8006-64-2 | 0.00 | 0.00 | 40.00 |
| HEXYL CINN ALD TBHQ | 101-86-0 | 0.00 | 5.10 | 0.00 |
| ISO BORNYL ACET | 125-12-2 | 182.86 | 80.00 | 50.00 |
| JAVAMOR TT (ELINCS) | 198404-98-7 | 0.00 | 0.00 | 4.00 |
| MANZANATE | 39255-32-8 | 0.00 | 0.00 | 35.00 |
| MELONAL TOCO | 106-72-9 | 0.00 | 0.00 | 30.00 |
| METH DH JASMONATE | 24851-98-7 | 0.00 | 35.00 | 0.00 |
| METH IONONE GAMMA "FF" TOCO | 127-51-5 | 0.00 | 0.00 | 150.00 |
| METH IONONE GAMMA A TOCO | 127-51-5 | 0.00 | 40.00 | 0.00 |
| METH PHEN ETH ETHER | 3558-60-9 | 0.00 | 0.00 | 3.00 |
| MONTAVERDI (ELINCS) | 188570-78-7 | 0.00 | 0.00 | 7.50 |
| ORANGE FLOWER ETHER TOCO | 14576-08-0 | 85.71 | 85.00 | 0.00 |
| PAMPLEZEST | 1465004-85-6 | 0.00 | 0.00 | 15.00 |
| PINO ACETALD TOCO | 33885-51-7 | 0.00 | 0.00 | 40.00 |
| ROSYRANE SUPER | 60335-71-9 | 0.00 | 0.00 | 30.00 |
| TERPINOLENE P UB TOCO | 586-62-9 | 57.14 | 130.00 | 0.00 |
| TETRAHYDRO LINALOOL | 78-69-3 | 22.76 | 70.00 | 39.00 |
| TETRAHYDRO LINALYL ACET | 68480-08-0 | 0.00 | 55.00 | 0.00 |
| TETRAHYDRO MYRCENOL | 18479-57-7 | 21.76 | 60.00 | 0.00 |
| | Total | 1000 | 1000 | 1000 |

### Example 19: Fragrance Performance with Encapsulated High Performance Fragrances

The aim of this example is to show the fragrance performance benefit of using encapsulated High Performance fragrances in fabric conditioner product. Fabric conditioner composition comprising fragrance compositions in Table 13 are washed with laundry and are evaluated by 12 trained panelists as described in Example 17. The results from the sensory panelists are then averaged.

The addition of encapsulated fragrance composition comprising High Performance fragrance ingredients of the present invention into a fabric conditioner base (*i*.*e*., High Performance Fragrance Examples 1 & 2), improves fragrance intensity at dry pre and dry gentle handling stages when compared to a benchmark encapsulated fragrance composition (*i*.*e*. Comparative Fragrance Example 1). Therefore, when using an encapsulated High Performance fragrance according to the present invention, fragrance intensity perception at dry pre and dry gentle handling stages can be further improved.

### Example 20: Exemplary Product Compositions

Composition A is an example of fine fragrance composition according to the present invention. It is prepared by admixture of the components described in Table 14, in the proportions indicated.

**TABLE 14**

| **Ingredient** | **Composition A (wt%*¹*)** |
|---|---|
| Ethanol SD-40 | 60-80 |
| Microcapsules *²* | 2.5-25 |
| DI-Water | Qs |
| Total | 100 |

| | |
|---|---|
| *¹* wt% relative to the total weight of the composition. *²* Encapsulated amount depends on the fragrance loading to 0.3% NOE. | |

Composition B is an example of fabric conditioner composition according to the present invention. It is prepared by admixture of the components described in Table 15, in the proportions indicated.

**TABLE 15**

| **Ingredient** | **Composition B (wt%*¹*)** |
|---|---|
| Example 17 fabric conditioner base | 90 |
| Microcapsules *²* | 0.5 - 3.0 |
| DI-Water | Qs |
| Total | 100 |

| | |
|---|---|
| *¹* wt% relative to the total weight of the composition. *²* Encapsulated amount depends on the fragrance loading to 0.2% NOE. | |

Composition C is an example of liquid detergent composition according to the present invention. It is prepared by admixture of the components described in Table 16, in the proportions indicated.

**TABLE 16**

| **Ingredient** | **Composition C (wt%*¹*)** |
|---|---|
| Black bull Liquid laundry detergent | 90 |
| Microcapsules *²* | 0.5-3.0 |
| DI-Water | Qs |
| Total | 100 |

| | |
|---|---|
| *¹* wt% relative to the total weight of the composition. *²* Encapsulated amount depends on the fragrance loading to 0.2% NOE. | |

Composition D is an example of powder detergent composition according to the present invention. It is prepared by admixture of the components described in Table 17, in the proportions indicated.

**TABLE 17**

| **Ingredient** | **Composition D (wt%*¹*)** |
|---|---|
| Linear alkylbenzenesulfonate | 22 |
| C₁₂₋₁₄ dimethylhydroxyethyl ammonium chloride | 0.2 |
| AE3S | 1 |
| Zeolite A | 1 |
| 1.6R silicate (SiO₂:Na₂O at ratio 1.6:1) | 5 |
| Sodium carbonate | 20 |
| Polyacrylate MW 4500 | 0.6 |
| Carboxymethyl cellulose | 0.3 |
| STAINZYME^{®} (20mg active/g) | 0.2 |
| Protease (SAVINASE^{®}, 32.89 mg active/g) | 0.1 |
| Lipase-LIPEX^{®} (18 mg active/g) | 0.07 |
| Fluorescent brightener | 0.06 |
| DTPA | 0.8 |
| MgSO₄ | 1 |
| Sodium percarbonate | 5.2 |
| TAED | 1.2 |
| Neat Fragrance | 0.5 |
| Microcapsules *²* | 0.5-3.0 |
| Total | 100 |

| | |
|---|---|
| *¹* wt% relative to the total weight of the composition. *²* Encapsulated amount depends on the fragrance loading to 0.2% NOE. | |

The present invention is not intended to be limited in scope to the particular disclosed embodiments, which are provided, for example, to illustrate various aspects of the invention. Various modifications to the compositions and methods described will become apparent from the description and teachings herein. Such variations may be practiced without departing from the true scope and spirit of the disclosure and are intended to fall within the scope of the present disclosure. Although the invention may be described in connection with specific preferred embodiments, it should be understood that the invention as claimed should not be unduly limited to such specific embodiments. Indeed, various modifications of the described modes for carrying out the invention which are obvious to those skilled in the art are intended to be within the scope of the following claims.

## Claims

1. Biodegradable core-shell microcapsules comprising:
(a) an isocyanate-biodegradable polymer shell comprising the reaction product of a biodegradable, isocyanate-terminated prepolymer with a crosslinker and optionally a polyelectrolyte emulsifier under aqueous conditions; and
(b) a core comprising an active material;
wherein the microcapsule shell has a biodegradation rate of at least 20%, 30%, 40%, 50%, 60%, 65%, 70%, 75%, 80%, 85%, 90%, 95% or 98%, within 60 days according to OECD301F or OECD310.

2. The biodegradable core-shell microcapsules of claim 1, wherein:
(a) the biodegradable, isocyanate-terminated prepolymer comprises gelatin, collagen, chitosan, modified guar, modified glucan, gum Arabic (gum acacia), modified gum Arabic (modified gum acacia), protein, hydrolyzed proteins, fermented proteins, hydrophobin, enzymes, partially neutralized citric acid ester, alginate, carrageenan, pectin, modified starch, or modified cellulose; and/or
(b) the crosslinker comprises an oxidized sugar, preferably the oxidized sugar comprises glucose, glucosamine, sucrose, maltose, lactose, maltodextrin, cyclodextrin, polysaccharide or a hydrolyzed polysaccharide.

3. The biodegradable core-shell microcapsules of claim 1 or 2, wherein:
(a) obtained slurry of the biodegradable core-shell microcapsules has a level of self-condensed polyisocyanate that is below 10%, below 5%, below 3%, below 1% or below 0.5%, relative to total weight of polyisocyanate used to form wall of the microcapsules; or
(b) materials used to form wall of the microcapsules do not form a blend of biodegradable materials and non-biodegradable materials; or
(c) materials used to form wall of the microcapsules forms a blend of biodegradable materials and non-biodegradable materials, wherein the levels of the non-biodegradable materials are below 10%, below 5%, below 3%, below 1% or below 0.5%, relative to weight of the microcapsules; or
(d) materials used to form the microcapsules forms a blend of biodegradable materials and non-biodegradable materials, wherein the biodegradation rate of all components of the blend is at least 20%, 30%, 40%, 50%, 60%, 65%, 70%, 75%, 80%, 85%, 90%, 95% or 98%, within 60 days according to OECD301F or OECD310.

4. The biodegradable core-shell microcapsules of any preceding claim, wherein the isocyanate-biodegradable polymer shell is highly crosslinked, preferably the average molecular weight of crosslinked prepolymer and polyelectrolyte emulsifier is at least 2, at least 4, or at least 10 of the average molecular weight of non-crosslinked prepolymer and polyelectrolyte emulsifier, as determined by size exclusion chromatography.

5. The biodegradable core-shell microcapsules of any preceding claim, wherein the polyelectrolyte emulsifier comprises gelatin, collagen, modified guar, modified glucan, gum Arabic (gum acacia), modified gum Arabic (modified gum acacia), protein, hydrolyzed proteins, fermented proteins, hydrophobin, enzymes, partially neutralized citric acid ester, alginate, carrageenan, pectin, modified starch, or modified cellulose.

6. The biodegradable core-shell microcapsules of any preceding claim, wherein the active material comprises a fragrance, flavor, agricultural active, pharmaceutical active, nutraceutical active, animal nutrition active, food active, microbio active, malodor counteractant, and/or cosmetic active, preferably a fragrance, more preferably a fragrance comprising at least one, at least two, at least three, at least four, at least five, at least six, at least seven, at least eight, at least nine or at least ten High Performance fragrance ingredients comprising Ultra High-Impact fragrance ingredients as listed in Table 1 and High-Impact fragrance ingredients as listed in Table 2, and optionally at least one additional fragrance ingredient.

7. A process for preparing biodegradable core-shell microcapsules comprising:
(a) reacting, under anhydrous conditions, a polyisocyanate dissolved in a solution with a biodegradable polymer, preferably in the presence of a catalyst, to form an isocyanate-terminated prepolymer, preferably the solution comprises (i) a water-soluble solvent, (ii) a water-soluble solvent comprising an active material or a portion thereof, or (iii) an active material, preferably the active material comprises a fragrance, flavor, agricultural active, pharmaceutical active, nutraceutical active, animal nutrition active, food active, microbio active, malodor counteractant, and/or cosmetic active, more preferably a fragrance;
(b) emulsifying the isocyanate-terminated prepolymer with an aqueous solution to form an emulsion, preferably said aqueous solution comprises a polyelectrolyte emulsifier; and
(c) crosslinking the isocyanate-terminated prepolymer to form biodegradable core-shell microcapsules;
preferably the microcapsule shell has a biodegradation rate of at least 20%, 30%, 40%, 50%, 60%, 65%, 70%, 75%, 80%, 85%, 90%, 95% or 98%, within 60 days according to OECD301F or OECD310.

8. The process of claim 7, wherein the biodegradable polymer is an amino-, hydroxyl- or carboxyl-containing biodegradable polymer, preferably the biodegradable polymer comprises gelatin, collagen, chitosan, modified guar, modified glucan, gum Arabic (gum acacia), modified gum Arabic (modified gum acacia), protein, hydrolyzed proteins, fermented proteins, hydrophobin, enzymes, partially neutralized citric acid ester, alginate, carrageenan, pectin, modified starch, or modified cellulose.

9. The process of claim 7, wherein the polyelectrolyte emulsifier comprises gelatin, collagen, modified guar, modified glucan, gum Arabic (gum acacia), modified gum Arabic (modified gum acacia), protein, hydrolyzed proteins, fermented proteins, hydrophobin, partially neutralized citric acid ester, alginate, carrageenan, pectin, modified starch, or modified cellulose.

10. The process of any one of claims 7 to 9, wherein the isocyanate-terminated prepolymer and/or the polyelectrolyte emulsifier is crosslinked with an oxidized sugar, preferably the sugar comprises glucose, glucosamine, sucrose, maltose, lactose, maltodextrin, cyclodextrin, polysaccharide or a hydrolyzed polysaccharide.

11. Biodegradable core-shell microcapsules obtainable by the process of any one of claims 7 to 10.

12. A consumer product comprising the biodegradable core-shell microcapsules of any one of claims 1 to 6, or claim 11, preferably the consumer product is a fabric softener, fabric conditioner, detergent, scent booster, fabric refresher spray, body wash, body soap, shampoo, hair conditioner, body spray, hair refresher spray, hair dye, hair moisturizer, skin moisturizer, hair treatment, skin treatment, candle, surface cleaner, bathroom cleaner, bleach, cat litter, refresher spray or pesticide.

13. A biodegradable, isocyanate-terminated prepolymer formed under anhydrous conditions in the presence of a catalyst for use in preparing core-shell microcapsules comprising the reaction product of:
(a) a polyisocyanate dissolved in an active material and optionally a solvent; and
(b) an amino-, hydroxyl- or carboxyl-containing biodegradable polymer, preferably the biodegradable polymer comprises gelatin, collagen, chitosan, modified guar, modified glucan, gum Arabic (gum acacia), modified gum Arabic (modified gum acacia), protein, hydrolyzed proteins, fermented proteins, hydrophobin, partially neutralized citric acid ester, alginate, carrageenan, pectin, modified starch, or modified cellulose;
wherein the prepolymer has a biodegradation rate of at least 20%, 30%, 40%, 50%, 60%, 65%, 70%, 75%, 80%, 85%, 90%, 95% or 98%, within 60 days according to OECD301F or OECD310.

14. A process for making a biodegradable, isocyanate-terminated prepolymer for use in preparing core-shell microcapsules comprising reacting
(a) a polyisocyanate dissolved in a solution, preferably the solution comprises (i) a water-soluble solvent, (ii) a water-soluble solvent comprising an active material or a portion thereof, or (iii) an active material, preferably the active material comprises a fragrance, flavor, agricultural active, pharmaceutical active, nutraceutical active, animal nutrition active, food active, microbio active, malodor counteractant, and/or cosmetic active, more preferably a fragrance; and
(b) a biodegradable polymer, preferably the biodegradable polymer is an amino-, hydroxyl- or carboxyl-containing biodegradable polymer, more preferably the biodegradable polymer comprises gelatin, collagen, chitosan, modified guar, modified glucan, gum Arabic (gum acacia), modified gum Arabic (modified gum acacia), protein, hydrolyzed proteins, fermented proteins, hydrophobin, partially neutralized citric acid ester, alginate, carrageenan, pectin, modified starch, or modified cellulose;
**characterized in that** reaction is carried out under anhydrous conditions in the presence of a catalyst.

15. A biodegradable, isocyanate-terminated prepolymer obtainable by the process of claim 14, preferably the biodegradable isocyanate-terminated biopolymer has a biodegradation rate of at least 20%, 30%, 40%, 50%, 60%, 65%, 70%, 75%, 80%, 85%, 90%, 95% or 98%, within 60 days according to OECD301F or OECD310.
